# EUROPÄISCHE PATENTSCHRIFT

(11) **EP 3 083 528 B1**
(45) Veröffentlichungstag und Bekanntmachung des Hinweises auf die Patenterteilung: **18.04.2018**
(21) Anmeldenummer: 14815284.6
(22) Anmeldetag: 09.12.2014
(51) Int. Cl.: C07C 2/46, C07C 7/04, C07C 13/277, C07C 2/42, C07C 7/148, B01J 31/40, B01J 31/22, B01J 31/14

(54) **VERFAHREN ZUR AUFARBEITUNG EINES REAKTIONSGEMISCHS (RG), DAS CYCLODODECATRIEN UND EIN AKTIVES KATALYSATORSYSTEM ENTHÄLT**
METHOD FOR PROCESSING A REACTION MIXTURE (RG) CONTAINING CYCLODODECATRIENE AND AN ACTIVE CATALYST SYSTEM
PROCÉDÉ POUR LE TRAITEMENT D'UN MÉLANGE RÉACTIONNEL (RG), QUI RENFERME UN CYCLODODÉCATRIÈNE ET UN SYSTÈME CATALYSEUR ACTIF

(30) Priorität: 11.12.2013 EP 13196667
(43) Veröffentlichungstag der Anmeldung: 26.10.2016
(73) Patentinhaber: BASF SE, 67056 Ludwigshafen am Rhein (DE)
(72) Erfinder: SCHWARZTRAUBER, Michael, 67434 Neustadt (DE); SCHELPER, Michael, Lane Bea, Discovery Bay (HK); GARCIA ANDARCIA, Hugo Rafael, Shanghai 200031 (CN); TURKSEVEN, Avni, 67105 Schifferstadt (DE); PACIELLO, Rocco, 67098 Bad Dürkheim (DE); SCHAUB, Thomas, 67433 Neustadt (DE)
(74) Vertreter: BASF IP Association
(86) Internationale Anmeldenummer: PCT/EP2014/077012
(87) Internationale Veröffentlichungsnummer: WO 2015/086578

(56) Entgegenhaltungen:
- DE-A1- 1 618 771
- DE-A1- 1 768 067
- US-A- 4 642 408

## Beschreibung

Die vorliegende Erfindung betrifft ein Verfahren zur Aufarbeitung eines Reaktionsgemischs (R_{G}), das Cyclododecatrien und ein aktives Katalysatorsystem enthält.

Cyclododecatrien wird nachfolgend als CDT abgekürzt. Entscheidet für die Qualität der aus CDT erhältlichen Folgeprodukte Cyclododecanon und Lauryllactam ist die Reinheit des eingesetzten Edukts CDT. Daher muss auf der Stufe des CDT darauf geachtet werden, dass Verbindungen wie Vinylcyclohexen, Cyclooctadien, C₁₆-Verbindungen, Oligomere und Polymer-Verbindungen möglichst weitgehend abgetrennt werden. In der Praxis ist zur Erzielung einer ausreichenden Produktqualität der Folgeprodukte Cyclododecanon und Lauryllactam in der Regel eine CDT-Reinheit von mehr als 99 % erforderlich. CDT wird durch Trimerisierung von Butadien hergestellt. Nebenprodukte der CDT-Herstellung sind Vinylcyclohexen, Cyclooctadien sowie Oligomere und Polymere des Butadiens mit 16, 20 oder mehr Kohlenstoffatomen. Darüber hinaus können bei der Herstellung, je nach verwendetem Katalysator, auch nahezu alle vorstehend beschriebenen Komponenten in einfach oder mehrfach chlorierter Form auftreten. Die cyclisierende Trimerisierung von Butadien zu Cyclododecatrien mit Hilfe von Ziegler Katalysatorsystemen besitzt großtechnische Bedeutung, da sie die cycloaliphatischen und offenkettigen C₁₂-Verbindungsklassen zugänglich macht. Interessante Folgeprodukte des CDT sind unter anderem Cyclododecanon, Cyclododecanol, Dekandicabonsäure und Lauryllactam. Laurinlactam ist von besonderem Interesse, da es ein Vorprodukt zur Herstellung von Nylon 12 darstellt.

Die Trimerisierung von Butadien zu Cyclododecatrien (CDT) erfolgt im Allgemeinen übergangsmetallkatalysiert, wobei beispielsweise Titan, Nickel oder Chromkatalysatoren eingesetzt werden, die mit einem Reduktionsmittel reduziert werden. Als Reduktionsmittel wird im Allgemeinen eine metallorganische Verbindung aus der ersten bis dritten Hauptgruppe des Periodensystems eingesetzt, wobei sich Aluminium-organische Verbindungen, wie beispielsweise Aluminiumalkyle, als vorteilhaft erwiesen haben. Für die industrielle Anwendung haben sich titanbasierte Katalysatoren wie Titantetrachlorid oder Titanacetylacetonat in Kombination mit Aluminiumalkylen als besonders vorteilhaft erwiesen.

Geeignete Katalysatorsysteme sind beispielsweise in US 3 878 258, US 3 655 795 und DE 3 021 840 beschrieben. Als Katalysatorsysteme werden bevorzugt solche eingesetzt, die Titantetrachlorid sowie Ethylaluminiumsesquichlorid oder Diethylaluminiumchlorid enthalten. Das Verhältnis von Al : Ti beträgt dabei im Allgemeinen 4 : 1 oder höher, um die Bildung von Polybutadien zu vermindern.

Typische Reaktionstemperaturen solcher Ziegler-Katalysatoren liegen im Allgemeinen im Bereich von 40 bis 90 °C. Beim Einsatz von Titan-Katalysatoren entsteht hauptsächlich das cis, trans, trans-Isomer des 1,5,9-Cyclododecatriens. Für den Fall, dass Nickel- oder Chrom-Katalysatoren eingesetzt werden, entsteht als Hauptprodukt das all-trans-Isomer des 1,5,9-Cyclododecatriens. Die Trimerisierungsreaktion des Butadiens kann in Gegenwart eines unpolaren inerten Lösungsmittels, wie beispielsweise Benzol, Cyclohexan, Hexan, Heptan, Octan, Decan, Toluol oder Xylol durchgeführt werden. Darüber hinaus ist auch eine Trimerisierung ohne Zugabe eines Lösungsmittels möglich. Solche Reaktionen sind beispielsweise in den Druckschriften DE 3 021 840 und US 6 403 851 beschreiben.

Die Reaktivität und Selektivität des Katalysatorsystems kann durch Zugabe von einem oder mehreren Promotoren modifiziert und verbessert werden. Geeignete Promotoren sind beispielsweise in den Druckschriften US 3 546 309, DE 2 825 341 und US 3 381 045 beschrieben.

Bei der Trimerisierung von Butadien zu Cyclododecatrien wird somit im Allgemeinen ein Reaktionsgemisch erhalten, das neben Cyclododecatrien und gegebenenfalls weiteren Nebenprodukten die vorstehend beschriebenen aktiven Katalysatorsysteme enthält. Zur weiteren Aufarbeitung des Zielprodukts Cyclododecatrien muss das aktive Katalysatorsystem entweder abgetrennt oder deaktiviert werden. Die weitere Aufarbeitung des Reaktionsgemischs erfolgt im Allgemeinen durch destillative Abtrennung des Cyclododecatriens. Würde die destillative Abtrennung des Cyclododecatrien in Gegenwart des aktiven Katalysators erfolgen, würde dies zu einer massiven Bildung von Nebenprodukten sowie zu einer teilweisen oder vollständigen Zerstörung des Cyclododecatriens führen.

In der DE 3 321 840 ist ein Verfahren beschrieben, bei dem das aktive Katalysatorsystem in Form eines Trägerkatalysators auf Polystyrol eingesetzt wird. Vor der destillativen Abtrennung des Cyclododecatriens wird der Trägerkatalysator abgetrennt. Dieses Verfahren ist jedoch aufwändig, da es ein zusätzlicher Abtrennungsschritt zwingend umfasst. Im Allgemeinen sind daher homogenkatalysierte Trimerisierungsreaktionen von Butadien bevorzugt. Die US-Patentschriften US 3,655,795 und US 3,878,259 setzen zur Deaktivierung des aktiven Katalysatorsystems gasförmigen Ammoniak ein. Das Reaktionsgemisch wird hierbei mit gasförmigem Ammoniak gesättigt.

Durch den Einsatz von gasförmigem Ammoniak wird das aktive Katalysatorsystem deaktiviert, so dass bei der destillativen Abtrennung des Cyclododecatriens die Bildung von Nebenprodukten bzw. die Zerstörung des Cyclododecatriens verhindert wird. Bei der Deaktivierung mit gasförmigem Ammoniak bilden sich aus den im aktiven Katalysatorsystem enthaltenen Aluminium-organischen Verbindungen, wie beispielsweise Ethylaluminiumsesquichlorid (Al₂Cl₃(C₂H₅)₃), allerdings verdampfbare Aluminiumverbindungen. Darüber hinaus kann es zur Bildung von Niederschlägen kommen, die vor der destillativen Aufarbeitung abfiltriert werden müssen.

Es wird vermutet, dass sich bei der Reaktion von Ammoniak mit den Aluminium-organischen Verbindungen Amido-Aluminiumchloride der empirischen Formeln AlCl₂NH₂ oder AlCl₃NH₃ bilden. Untersuchungen hierzu sind beispielsweise in J. Chem. Soc. 1965, S. 1092 bis 1096 und J. Am. Chem. Soc. 1960, 83, S. 542 bis 546 beischrieben. Diese Amido-Aluminiumchloride werden bei der destillativen Abtrennung des Cyclododecatriens verdampft und führen somit bei der Aufarbeitung des Cyclododecatriens zu Problemen, da sie beispielsweise in Wärmetauschern Ablagerungen bilden, die sich nachfolgend nur mit sehr hohem Reinigungsaufwand wieder entfernen lassen. Es wird vermutet, dass die Amido-Aluminiumchloride nach bzw. beim Verdampfen untereinander eine Kondensationsreaktion eingehen, wobei sich polymere Kondensationsprodukte bilden, die für die Ablagerungen verantwortlich sind. In der J. Chem. Soc. 1965, S. 1092 bis 1096 und J. Am. Chem. Soc. 1960, 83, S. 542 bis 546 wird vermutet, dass die Kondensationsprodukte (Ablagerungen) die folgenden empirischen Formeln aufweisen; (AlN)ₓ, bzw. (AlClNH)ₓ. Zudem kann es durch Anwesenheit von Wasser in späteren Aufarbeitungsschritten (z.B. Wäsche des Rohgemisches nach der Verdampfung) zur Hydrolyse dieser Amido-Aluminiumchloride unter Bildung von unlöslichem Aluminiumoxid kommen. Auch dies kann zu unerwünschten Ablagerungen führen, welche zusätzlichen Reinigungsaufwand in der Anlage nötig machen.

Die US 3,381,045 und US 3,546,309 beschreiben den Einsatz von Isopropylalkohol bzw. Aceton zur Deaktivierung des aktiven Katalysatorsystems. Hierdurch werden die Bildung von verdampfbaren Amido-Aluminiumchloriden und die damit verbundene Bildung von Ablagerungen vermieden. Allerdings bilden sich bei dem Einsatz von polaren Lösungsmitteln wie Alkoholen zwei Flüssigkeitsphasen aus, die vor der destillativen Aufarbeitung getrennt werden müssen.

Außerdem kommt es beim Einsatz von polaren Lösungsmitteln wie Isopropylalkohol oder Aceton zur Bildung von Niederschlägen sowie zur Mulmbildung. Diese erschwert die weitere Aufarbeitung des Cyclododecatriens. Die Niederschläge müssen abfiltriert werden. Die Mulmbindung erschwert zudem die Phasentrennung der beiden Flüssigphasen. Mulm, auch als Lockersediment bezeichnet, bildet sich bei diesen Deaktivierungsmethoden zwischen den beiden Flüssigphasen als dritte Phase, die sowohl Flüssigkeit als auch Feststoffe enthält. Eine Aufarbeitung des Reaktionsgemischs nach Deaktivierung des aktiven Katalysatorsystems ist nach den in der US 3,381,045 und US 3,546,309 beschriebenen Verfahren großtechnisch daher nur äußerst schwierig realisierbar. Der Einsatz von polaren Lösungsmitteln wie Alkoholen oder Aceton zur Deaktivierung des aktiven Katalysatorsystems hat zudem den Nachteil, dass sie die Bildung chlorierter Nebenprodukte begünstigen, was sich negativ auf die Produktqualität des Cyclododecatriens auswirkt und die Gewinnung eines spezifikationsgerechten Cyclododecatriens erschwert.

Die DE 1 768 067 beschreibt ein Verfahren zur Aufarbeitung von Reaktionsgemischen, die Cyclododecatrien enthalten, wobei zur Deaktivierung des aktiven Katalysatorsystems eine konzentrierte wässrige Ammoniaklösung eingesetzt wird. Bevorzugt wird nach Zugabe der konzentrierten wässrigen Ammoniaklösung nachfolgend noch zusätzlich Wasser oder eine 20 %-ige wässrige Natronlauge zugegeben. Auch bei dem Aufarbeitungsverfahren gemäß DE 1 768 067 bildet sich bei der Zugabe der konzentrierten wässrigen Ammoniaklösung ein Niederschlag aus, der abfiltriert werden muss. Durch die bevorzugte Zugabe von Wasser wird die Bildung des Niederschlags noch begünstigt. Für den Fall, dass man nach Zugabe der konzentrierten wässrigen Ammoniaklösung nachfolgend eine wässrige Natronlauge zugibt, löst sich der Niederschlag wieder auf, wobei sich jedoch in jedem Fall zwei Flüssigkeitsphasen bilden, die getrennt werden müssen. Auch bei dem Verfahren gemäß DE 1 768 067 ist somit das Abfiltrieren des gebildeten Niederschlags beziehungsweise eine Phasentrennung der zwei gebildeten Flüssigphasen zwingend erforderlich.

Die im Stand der Technik beschriebenen Verfahren zur Aufarbeitung von Reaktionsgemischen, die Cyclododecatrien und ein aktives Katalysatorsystem enthalten, sind daher großtechnisch schwer realisierbar. Dies liegt darin begründet, dass bei diesen Verfahren das Abfiltrieren des gebildeten Niederschlages oder eine Phasentrennung der beiden gebildeten Flüssigphasen zwingend erforderlich ist. Die im Stand der Technik beschriebenen Verfahren, bei denen gasförmiger Ammoniak zur Deaktivierung des aktiven Katalysatorsystems eingesetzt wird, zeigen zudem den Nachteil, dass bei der nachfolgenden destillativen Abtrennung des Cyclododecatriens verdampfbare Amido-Aluminiumchloride zu Ablagerungen führen, die eine kontinuierliche destillative Abtrennung des Cyclododecatriens erschweren.

Der vorliegenden Erfindung liegt somit die Aufgabe zugrunde, ein Verfahren bereitzustellen, dass die Nachteile des Standes der Technik nicht oder nur in vermindertem Maße aufweist. Das Verfahren soll insbesondere die Aufarbeitung eines Reaktionsgemischs, das Cyclododecatrien und ein aktives Katalysatorsystem enthält ermöglichen, ohne dass eine Phasentrennung von zwei Flüssigphasen notwendig wird. Darüber hinaus soll das Abfiltrieren von Niederschlägen weitestgehend vermieden werden. Außerdem soll die Bildung von Ablagerungen, die bei der destillativen Aufarbeitung des Cyclododecatriens durch verdampfbare Amido-Aluminiumchloride hervorgerufen werden, verhindert werden. Das erfindungsgemäße Verfahren soll zudem kostengünstig, wirtschaftlich und mit einer gegenüber den im Stand der Technik beschriebenen Verfahren verringerten Anzahl von Verfahrensschritten durchführbar sein.

Gelöst wird diese Aufgabe durch ein Verfahren zur Aufarbeitung eines Reaktionsgemischs (R_{G}) enthaltend Cyclododecatrien und ein aktives Katalysatorsystem (K), das eine Aluminium-organische Verbindung enthält, umfassend die Schritte:
a) Inkontaktbringen des Reaktionsgemischs (R_{G}) mit gasförmigem Ammoniak unter Erhalt eines ersten Gemischs (G1),
b) Inkontaktbringen des ersten Gemischs (G1) mit Wasser unter Erhalt eines zweiten Gemischs (G2),
c) Destillative Abtrennung von Cyclododecatrien aus dem zweiten Gemisch (G2).

Es wurde überraschenderweise gefunden, dass das erfindungsgemäße Verfahren eine verbesserte und insbesondere wirtschaftlichere Aufarbeitung von Reaktionsgemischen (R_{G}) ermöglicht, die Cyclododecatrien und ein aktives Katalysatorsystem (K) enthalten, dass eine Aluminium-organische Verbindung enthält. Bei dem erfindungsgemäßen Verfahren ist nach der Deaktivierung des aktiven Katalysatorsystems (K) eine Phasentrennung nicht erforderlich, da das in Verfahrensschritt b) erhaltene zweite Gemisch (G2) bevorzugt nur eine Flüssigphase enthält. Das erfindungsgemäße Verfahren hat zudem den Vorteil, dass durch das Inkontaktbringen des ersten Gemischs (G1) mit Wasser gemäß Verfahrensschritt b) verdampfbare Amido-Aluminiumchloride sicher zerstört werden, so dass es bei der destillativen Abtrennung des Cyclododecatriens gemäß Verfahrensschritt c) nicht zur Bildung von Ablagerungen kommt. Darüber hinaus bildet sich beim erfindungsgemäßen Verfahren kein oder nur sehr wenig Niederschlag, der abfiltriert werden muss. Dementsprechend müssen die eingesetzten Filter nur selten gewechselt werden, was den kontinuierlichen Betrieb des erfindungsgemäßen Verfahrens begünstigt. Der Großteil des zweiten deaktivierten Katalysatorsystems (K2) verbleibt nach dem erfindungsgemäßen Verfahren überraschenderweise in der Flüssigphase und kann nach der destillativen Abtrennung des Cyclododecatriens leicht als Sumpf der Destillationsvorrichtung entsorgt werden.

### Reaktionsgemisch (R_{G})

Das Reaktionsgemisch (R_{G}) enthält Cyclododecatrien und ein aktives Katalysatorsystem (K), das eine Aluminium-organische Verbindung enthält. Das Reaktionsgemisch (R_{G}) entstammt im Allgemeinen einer homogen-katalysierten Trimerisierungsreaktion von Butadien.

Unter Cyclododecatrien werden erfindungsgemäß sämtliche Isomere des Cyclododecatriens verstanden. Cyclododecatrien, genauer 1,5,9-Cyclododecatrien, kann in vier unterschiedlichen isomeren Formen vorliegen.

Diese sind Z,Z,Z-1,5,9-Cyclododecatrien (CAS-Nr. 4736-48-5), E,E,E-1,5,9-Cyclododecatrien (CAS-Nr. 676-22-2), E,Z,Z-1,5,9-Cyclododecatrien (CAS-Nr. 2765-29-9) und E,E,Z-1,5,9-Cyclododecatrien (CAS-Nr. 706-31-0).

Bei der Trimerisierung (Cyclotrimerisierung) von 1,3-Butadien zu Cyclododecatrien bilden sich im Allgemeinen Gemische der vorstehend genannten Isomere. In Abhängigkeit des eingesetzten aktiven Katalysatorsystems (K) kann das Verhältnis der Isomere zueinander gesteuert werden. So entsteht beispielsweise bei Einsatz von Nickel- beziehungsweise Chrom- haltigen Katalysatorsystemen (K) überwiegend das Z,Z,Z-1,5,9-Cyclododecatrien, wohingegen sich beim Einsatz von Titan-haltigen aktiven Katalysatorsystemen (K) hauptsächlich das E,Z,Z-1,5,9-Cyclododecatrien bildet. Dieses wird auch als 1,5,9-*trans-trans-cis*-Cyclododecatrien bezeichnet. Die Art der im Reaktionsgemisch (R_{G}) enthaltenen Isomere ist nicht erfindungswesentlich.

Bevorzugt enthält das Reaktionsgemisch (R_{G}) jedoch hauptsächlich das E,E,Z-1,5,9-Cyclododecatrien. In einer besonders bevorzugten Ausführungsform enthält das Reaktionsgemisch (R_{G}) mindestens 80 Gew.-%, bevorzugt mindestens 90 Gew.-% des E,E,Z-1,5,9-Cyclododecatriens, bezogen auf das Gesamtgewicht aller Cyclododecatrien-Isomere, die im Reaktionsgemisch (R_{G}) enthalten sind.

Das Reaktionsgemisch (R_{G}) entstammt bevorzugt einer Trimerisierungsreaktion von 1,3-Butadien in Gegenwart eines aktiven Katalysatorsystems (K), das eine Aluminium-organische Verbindung sowie eine Titanverbindung der Oxidationsstufe +IV enthält. Das Reaktionsgemisch (R_{G}) enthält besonders bevorzugt ein aktives Katalysatorsystem (K), das aus mindestens einer Aluminium-organischen Verbindung ausgewählt aus der Gruppe bestehend aus Al₂(C₂H₅)₆, Al₂Cl₃(C₂H₅)₃ und AlCl(C₂H₅)₂ und mindestens einer Titanverbindung, ausgewählt aus der Gruppe bestehend aus Titanacetylacetonat und Titantetrachlorid, erhältlich ist. Insbesondere bevorzugt enthält das Reaktionsgemisch (R_{G}) ein aktives Katalysatorsystem, das aus Titantetrachlorid (TiCl₄) und Ethylaluminiumsesquichlorid Al₂Cl₃(C₂H₅)₃ gebildet wird.

Zur Trimerisierung von 1,3-Butadien zu 1,5,9-Cyclododecatrien werden im Allgemeinen pro 1 mol 1,3-Butadien, 0,0001 bis 0,1 mol einer Aluminium-organischen Verbindung, 0,00001 bis 0,01 mol einer Titanverbindung der Oxidationsstufe +IV sowie gegebenenfalls 0,00001 bis 0,01 mol eines Promotors eingesetzt.

Für den Fall, dass ein Promotor eingesetzt wird, ist Wasser als Promotor besonders bevorzugt.

Zur Erzeugung des aktiven Katalysatorsystems (K) werden bevorzugt pro 1 mol der Titanverbindung der Oxidationsstufe +IV, 2 bis 50 mol der Aluminium-organischen Verbindung und 0,1 bis 20 mol des Promotors, bevorzugt des Wassers, eingesetzt.

Die Trimerisierung des 1,3-Butadiens kann bevorzugt in Gegenwart eines unpolaren Lösungsmittels durchgeführt werden. Darüber hinaus ist es auch möglich, die Trimerisierung in Abwesenheit eines solchen unpolaren Lösungsmittels durchzuführen. Als unpolare Lösungsmittel sind Lösungsmittel besonders bevorzugt, die unter den Reaktionsbedingungen der Trimerisierung von 1,3-Butadien inert sind. Unter "inert" wird erfindungsgemäß verstanden, dass die inerten unpolaren Lösungsmittel unter den Reaktionsbedingungen der Trimerisierung von 1,3-Butadien chemisch nicht verändert werden.

Besonders bevorzugt ist ein Reaktionsgemisch (R_{G}), bei dem die Trimerisierung in Gegenwart eines unpolaren Lösungsmittels durchgeführt wird. Als unpolare (inerte) Lösungsmittel sind beispielsweise mindestens ein Lösungsmittel, ausgewählt aus der Gruppe bestehend aus Benzol, Cyclohexan, Hexan, Heptan, Octan, Decan, Toluol und Xylol geeignet. Hexan, Heptan, Octan, Decan sowie Xylol umfassen erfindungsgemäß alle Isomere dieser Verbindungen. Bevorzugt ist ein Reaktionsgemisch (R_{G}), bei dem die Trimerisierung in Gegenwart von Toluol durchgeführt wird. Unter den unpolaren (inerten) Lösungsmitteln ist somit Toluol insbesondere bevorzugt.

Gegenstand der vorliegenden Erfindung ist somit auch ein Verfahren, bei dem das mindestens eine unpolare Lösungsmittel ausgewählt ist aus der Gruppe bestehend aus Benzol, Cyclohexan, Hexan, Heptan, Octan, Decan und Xylol.

Besonders bevorzugt entstammt das Reaktionsgemisch (R_{G}) einer Trimerisierungsreaktion von 1,3-Butadien, wie sie in der WO 2009092683 beschrieben ist.

Besonders bevorzugte Reaktionsgemische (R_{G}) enthalten somit 15 bis 70 Gew.-% Cyclododecatrien, 10 bis 80 Gew.-% mindestens eines unpolaren Lösungsmittels und 0,01 bis 5 Gew.-% des aktiven Katalysatorsystems (K), wobei die Gew.-%-Angaben jeweils auf das Gesamtgewicht des Reaktionsgemischs (R_{G}) bezogen sind. Im Hinblick auf das vorstehend beschriebene Reaktionsgemisch (R_{G}) gelten die Ausführungen und Bevorzugungen im Hinblick auf Cyclododecatrien, das unpolare Lösungsmittel sowie das aktive Katalysatorsystem entsprechend.

Gegenstand der vorliegenden Erfindung ist somit auch ein Verfahren bei dem dem Reaktionsgemisch (R_{G}) in Verfahrensschritt a) pro 1 kg des Reaktionsgemischs (R_{G}) 0,1 bis 20 g gasförmiger Ammoniak zugegeben wird.

Die Summe der Gew.-%-Angaben zum Reaktionsgemisch (R_{G}) ergibt 100 Gew.-%

Gegenstand der vorliegenden Erfindung ist somit auch ein Verfahren, bei dem das aktive Katalysatorsystem (K) mindestens eine Aluminium-organische Verbindung ausgewählt aus der Gruppe bestehend aus Al₂(C₂H₅)₆, Al₂Cl₃(C₂H₅)₃ und AlCl(C₂H₅)₂ sowie mindestens eine Titanverbindung ausgewählt aus der Gruppe bestehend aus Titantetrachlorid und Titanacetylacetonat enthält.

Als aktives Katalysatorsystem (K) enthält das Reaktionsgemisch (R_{G}) insbesondere eine Katalysatorsystem (K), das aus Titantetrachlorid und Ethylaluminiumsesquichlorid (Al₂Cl₃(C₂H₅)₃) und Wasser erhältlich ist, wobei pro 1 mol Titantetrachlorid 2 bis 50 mol Ethylaluminiumsesquichlorid und 0.1 bis 20 mol Wasser eingesetzt werden.

### Verfahrensschritt a)

Gemäß Verfahrensschritt a) wird das vorstehend beschriebene Reaktionsgemisch (R_{G}) mit gasförmigem Ammoniak in Kontakt gebracht. Hierdurch wird ein erstes Gemisch (G1) erhalten, das Cyclododecatrien und ein erstes deaktiviertes Katalysatorsystem (K1) enthält.

Es wird vermutet, dass die im Reaktionsgemisch (R_{G}) enthaltene Aluminium-organische Verbindung durch das Inkontaktbringen mit gasförmigem Ammoniak in Amido-Aluminiumchloride, wie sie im einleitenden Teil der Beschreibung der vorliegenden Erfindung beschrieben sind, umgewandelt wird. Hierdurch wird das aktive Katalysatorsystem (K) in das erste deaktivierte Katalysatorsystem (K1) umgewandelt. Das erste Gemisch (G1) enthält somit Aluminiumverbindungen, die bei 400 °C einen Dampfdruck von größer 1000 mbar aufweisen.

Die Bestimmung des Dampfdrucks erfolgt erfindungsgemäß nach der "OECD Guidline for the Testing of Chemicals; 104" vom 27. Juli 1995.

Gegenstand der vorliegenden Erfindung ist somit auch ein Verfahren, bei dem das erste Gemisch (G1) Cyclododecatrien und ein erstes deaktivierte Katalysatorsystem (K1) enthält, wobei das erste Gemisch (G1) Aluminiumverbindungen enthält, die bei 400°C einen Dampfdruck von größer 1000 mbar aufweisen.

Es wird vermutet, dass es sich bei den Aluminiumverbindungen, die bei 400 °C einen Dampfdruck von größer 1000 mbar aufweisen, um die einleitend beschriebenen Amido-Aluminiumchloride handelt. Die vorstehend beschriebenen Vermutungen sollen die vorliegende Erfindung nicht beschränken.

Die Aluminiumverbindungen, die bei 400 °C einen Dampfdruck von größer 1000 mbar aufweisen, würden bei einer direkten destillativen Abtrennung von Cyclododecatrien aus dem ersten Gemisch (G1) mit verdampft werden und in der Destillationsvorrichtung zu Ablagerungen führen. Es wird vermutet, dass sich unter den Bedingungen der destillativen Abtrennung von Cyclododecatrien aus dem ersten Gemisch (G1), wie es in den Verfahren im Stand der Technik beschrieben ist, polymere Aluminiumverbindungen bilden. Es wird vermutet, dass die verdampfbaren Amido-Aluminiumchloride unter Abspaltung von Ammoniak beziehungsweise Chlorwasserstoff zu polymeren Verbindungen der empirischen Formeln (AlN)ₓ bzw. (AlClNH)ₓ kondensieren oder in weiteren Schritten der destilaltiven Aufarbeitung zu unlöslichen Verbindungen hydrolysieren. Diese Kondensationsprodukte (Ablagerungen) sind in organischen Lösungsmitteln nicht löslich und können nur unter großem mechanischem Aufwand aus der Destillationsvorrichtung entfernt werden.

Das Inkontaktbringen des Reaktionsgemischs (R_{G}) mit gasförmigem Ammoniak kann bei Temperaturen im Bereich von 20 bis 140 °C, bevorzugt im Bereich von 30 bis 90 °C und bei einem Druck im Bereich von 0,5 bar_{abs} bis 50 bar_{abs}, bevorzugt im Bereich zwischen 1 bar_{abs} und 5 bar_{abs} durchgeführt werden.

Der gasförmige Ammoniak ist bevorzugt wasserfrei. Unter "wasserfrei" wird vorliegend verstanden, dass der gasförmige Ammoniak weniger als 1 Gew.-%, bevorzugt weniger als 0,5 Gew-%, besonders bevorzugt weniger als 0,1 Gew.-% Wasser enthält, jeweils bezogen auf das Gesamtgewicht des in Verfahrensschritt a) eingesetzten gasförmigen Ammoniaks.

In Verfahrensschritt a) wird das Reaktionsgemisch (R_{G}) im Allgemeinen mit 5 bis 20 g, bevorzugt mit 1 bis 5 g gasförmigem Ammoniak in Kontakt gebracht, jeweils bezogen auf 1 kg des Reaktionsgemischs (R_{G}). In einer bevorzugten Ausführungsform werden dem Reaktionsgemisch (R_{G}) pro 1 kg Reaktionsgemisch (R_{G}) im Allgemeinen 0.5 bis 5 g, bevorzugt 1 bis 3 g gasförmiger Ammoniak zugegeben.

Gegenstand der vorliegenden Erfindung ist somit auch ein Verfahren, bei dem dem Reaktionsgemisch (R_{G}) in Verfahrensschritt a) pro 1 kg des Reaktionsgemischs (R_{G}) 0,1 bis 20 g gasförmiger Ammoniak zugegeben werden.

In einer bevorzugten Ausführungsform werden dem Reaktionsgemisch (R_{G}) pro 1 mol der im Reaktionsgemisch (R_{G}) enthaltenen Aluminium-organischen Verbindung 0,1 bis 50 mol, bevorzugt 1 bis 40 mol gasförmiger Ammoniak zugegeben.

Verfahrensschritt a) wird bevorzugt im kontinuierlichen Betrieb durchgeführt. Geeignete Vorrichtungen zum Inkontaktbringen des Reaktionsgemischs (R_{G}) mit gasförmigem Ammoniak sind beispielsweise Rührkessel, Rührkesselkaskaden oder Rohrreaktoren. Das Inkontaktbringen des Reaktionsgemischs (R_{G}) mit gasförmigem Ammoniak wird bevorzugt für eine Zeitdauer von mindestens 0,1 Stunden, besonders bevorzugt von mindestens 0,3 Stunden, insbesondere bevorzugt von mindestens 0,5 Stunden und am meisten bevorzugt von mindestens 1 Stunde durchgeführt. Das Inkontaktbringen kann gemäß Verfahrensschritt a) in beliebig langen Zeiträumen durchgeführt werden. Im Allgemeinen beträgt die Zeitdauer jedoch maximal 24 Stunden. Bevorzugt wird Verfahrensschritt a) somit für eine Zeitdauer im Bereich von 0,1 bis 24 Stunden, besonders bevorzugt für eine Zeitdauer im Bereich von 0,3 bis 12 Stunden und insbesondere bevorzugt für eine Zeitdauer im Bereich von 0.5 bis 5 Stunden durchgeführt.

Gegenstand der vorliegenden Erfindung ist somit auch ein Verfahren, bei dem Verfahrensschritt a) für eine Zeitdauer von mindestens 0,1 Stunden durchgeführt wird.

Die Zeitdauer beschreibt somit die Dauer des Verfahrensschritts a), das heißt die Zeitdauer vom ersten Inkontaktbringen des Reaktionsgemischs (R_{G}) mit gasförmigem Ammoniak bis zum Inkontaktbringen des ersten Gemischs (G1) mit Wasser gemäß Verfahrensschritt b).

Während Verfahrensschritt a) bildet das erste Gemisch (G1) nur eine Flüssigphase aus.

Gegenstand der vorliegenden Erfindung ist somit auch ein Verfahren, bei dem das erste Gemisch (G1) nur eine Flüssigkeitsphase aufweist.

Die Ausbildung von zwei Flüssigphasen, wie sie bei den im Stand der Technik beschriebenen Verfahren auftritt, wird durch das erfindungsgemäße Verfahren vermieden. Darüber hinaus bildet sich in Verfahrensschritt a) im Wesentlichen kein Niederschlag aus. Unter "im Wesentlichen kein Niederschlag" wird erfindungsgemäß verstanden, dass maximal 1 Gew.-%, bevorzugt maximal 0,5 Gew.-% und insbesondere bevorzugt maximal 0,25 Gew.-% Feststoff ausfallen, jeweils bezogen auf das Gesamtgewicht des ersten Gemischs (G1). Hierdurch kann das erste Gemisch (G1) in einer bevorzugten Ausführungsform ohne einen Phasentrennungsschritt und ohne einen Filtrationsschritt direkt Verfahrensschritt b) zugeführt werden.

Gegenstand der vorliegenden Erfindung ist somit auch ein Verfahren, bei dem das erste Gemisch (G1) weniger als 1 Gew.-% Feststoff enthält, bezogen auf das Gesamtgewicht des ersten Gemischs (G1).

### Verfahrensschritt b)

Gemäß Verfahrensschritt b) wird das in Verfahrensschritt a) erhaltene erste Gemisch (G1) mit Wasser in Kontakt gebracht. Hierdurch werden die im ersten Gemisch (G1) enthaltenen Aluminiumverbindungen, die bei 400 °C einen Dampfdruck von größer 1000 mbar aufweisen, unter Erhalt des zweiten deaktivierten Katalysatorsystems (K2) zersetzt. Das zweite Gemisch (G2) enthält somit keine Aluminiumverbindungen, die bei 400 °C einen Dampfdruck von größer 1000 mbar aufweisen.

Gegenstand der vorliegenden Erfindung ist somit auch ein Verfahren, bei dem das zweite Gemisch (G2) Cyclododecatrien und ein zweites deaktiviertes Katalysatorsystem (K2) enthält, wobei das zweite Gemisch (G2) keine Aluminiumverbindungen enthält, die bei 400°C einen Dampfdruck von größer 1000 mbar aufweisen.

Es wird vermutet, dass sich durch die Zugabe von Wasser die im ersten Gemisch (G1) enthaltenen verdampfbaren Amido-Aluminiumchloride in nicht verdampfbaren Aluminiumsauerstoffverbindungen umwandeln.

Gemäß Verfahrensschritt b) wird das in Verfahrensschritt a) erhaltene erste Gemisch (G1) gerade mit so viel Wasser in Kontakt gebracht, dass sich das Wasser vollständig in dem erhaltenen zweiten Gemisch (G2) löst.

Bevorzugt wird nur so viel Wasser in Verfahrensschritt b) zugegeben, dass das zweite Gemisch (G2) nur eine Flüssigphase enthält. Hierdurch wird der Phasentrennungsschritt, der bei den im Stand der Technik beschriebenen Verfahren zwingend erforderlich ist, eingespart. In einer bevorzugten Ausführungsform der vorliegenden Erfindung wird das zweite Gemisch (G2) ohne einen vorherigen Phasentrennungsschritt, das heißt die Trennung von zwei Flüssigphasen, der destillativen Abtrennung von Cyclododecatrien aus dem zweiten Gemisch (G2) gemäß Verfahrensschritt c) zugeführt.

In einer bevorzugten Ausführungsform werden dem ersten Gemisch (G1) pro 1 kg des ersten Gemischs (G1) 0,05 bis 1,0 g, besonders bevorzugt 0,05 g bis 0,5 g und insbesondere bevorzugt 0,05 g bis 0,2 g Wasser zugegeben.

Gegenstand der vorliegenden Erfindung ist somit auch ein Verfahren, bei dem dem ersten Gemisch (G1) in Verfahrensschritt b) pro 1 kg des ersten Gemischs (G1) 0,05 bis 1,0 g Wasser zugegeben werden.

In einer bevorzugten Ausführungsform werden dem ersten Gemisch (G1) pro 1 mol der ursprünglich im Reaktionsgemisch (R_{G}) enthaltenen Aluminium-organischen Verbindung(en) 0,1 bis 10 mol, besonders bevorzugt 0,5 bis 2 mol Wasser zugegeben.

Das Inkontaktbringen mit Wasser gemäß Verfahrensschritt b) unter Erhalt des zweiten Gemischs (G2) wird bevorzugt für eine Zeitdauer von mindestens 0,5 Stunden, bevorzugt mindestens 1 Stunde und besonders bevorzugt mindestens 2 Stunden, durchgeführt.

Das Inkontaktbringen des ersten Gemischs (G1) mit Wasser kann gemäß Verfahrensschritt b) in beliebig langen Zeiträumen durchgeführt werden. Im Allgemeinen beträgt die Zeitdauer jedoch maximal 24 Stunden. Bevorzugt wird Verfahrensschritt b) für eine Zeitdauer von 0,5 bis 24 Stunden, besonders bevorzugt 1 bis 20 Stunden und insbesondere bevorzugt 2 bis 18 Stunden durchgeführt.

Gegenstand der vorliegenden Erfindung ist somit auch ein Verfahren, bei dem Verfahrensschritt b) für eine Zeitdauer von mindestens 0,5 Stunden durchgeführt wird.

Das Inkontaktbringen gemäß Verfahrensschritt b) kann bei Temperaturen im Bereich von 20 bis 100 °C und bei Drücken im Bereich von 0,5 bar_{abs} bis 10 bar_{abs} durchgeführt werden. Bevorzugt wird Verfahrensschritt b) in einer Verweilzeitvorrichtung durchgeführt. Hierbei kann es sich um einen kontinuierlich betriebenen Rührkessel oder um eine kontinuierlich betriebene Rührkesselkaskade handeln. Bevorzugt als Verweilzeitvorrichtung ist jedoch ein Rohrbündelreaktor. Das Inkontaktbringen mit Wasser gemäß Verfahrensschritt b) kann hierbei in der Verweilzeitvorrichtung erfolgen. Bevorzugt wird das Wasser dem ersten Gemisch (G1) jedoch vor Eintritt in die Verweilzeitvorrichtung zugegeben.

Während beziehungsweise nach Verfahrensschritt b) wird ein zweites Gemisch (G2) erhalten, das ein zweites deaktiviertes Katalysatorsystem (K2) enthält, wobei das zweite Gemisch (G2) keine Aluminiumverbindungen enthält, die bei 400 °C einen Dampfdruck größer 1000 mbar aufweisen.

Hierdurch wird die Bildung von Ablagerungen bei der destillativen Abtrennung von Cyclododecatrien aus dem zweiten Gemisch (G2) in der Destillationsvorrichtung gemäß Verfahrensschritt c) wirksam verhindert.

Das zweite Gemisch (G2) weist bevorzugt nur eine Flüssigkeitsphase auf.

Gegenstand der vorliegenden Erfindung ist somit auch ein Verfahren, bei dem das zweite Gemisch (G2) nur eine Flüssigphase enthält.

Bevorzugt wird das in Verfahrensschritt b) erhaltene zweite Gemisch (G2) ohne die Durchführung eines Flüssigkeits-Flüssigkeits-Phasentrennschritts dem Verfahrensschritt c) zugeführt. Unter "Phasentrennschritt" wird erfindungsgemäß die Trennung von zwei Flüssigkeitsphasen verstanden.

Überraschenderweise fallen während Verfahrensschritt b) zudem keine oder nur geringe Mengen an Niederschlag (Feststoff) aus. Bevorzugt enthält das zweite Gemisch (G2) weniger als 10 Gew.-% Feststoff, besonders bevorzugt weniger als 5 Gew.-% und insbesondere weniger als 1 Gew.-% Feststoff, jeweils bezogen auf das Gesamtgewicht des zweiten Gemischs (G2).

Gegenstand der vorliegenden Erfindung ist somit auch ein Verfahren, bei dem das zweite Gemisch (G2) weniger als 10 Gew.-% Feststoff enthält, bezogen auf das Gesamtgewicht des zweiten Gemischs (G2).

Das zweite Gemisch (G2) kann in einer Ausführungsform ohne einen Filtrationsschritt dem Verfahrensschritt c) zugeführt werden. Bevorzugt wird das zweite Gemisch (G2) vor Verfahrensschritt c) einem Filtrationsschritt unterzogen, um gegebenenfalls im zweiten Gemisch (G2) enthaltenen Feststoff abzutrennen.

### Verfahrensschritt c)

In Verfahrensschritt c) wird das Cyclododecatrien aus dem zweiten Gemisch (G2) destillativ abgetrennt.

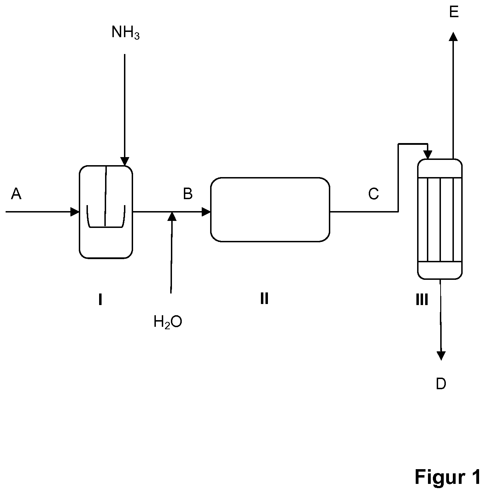

Die destillative Abtrennung kann bei Temperaturen im Bereich von 140 bis 220 °C und bei Drücken im Bereich von 10 mbar_{abs} bis 1 bar_{abs} durchgeführt werden. Bevorzugt ist eine destillative Abtrennung wie sie beispielsweise in der WO 2009092682 beschrieben ist. Geeignete Destillationsvorrichtungen sind beispielsweise Destillationskolonnen oder Verdampfer, wobei Verdampfer bevorzugt sind. Über Kopf wird in Verfahrensschritt c) das Zielprodukt Cyclododecatrien, sowie gegebenenfalls enthaltene unpolare Lösungsmittel, bevorzugt Toluol, und gegebenenfalls weitere Leichtsieder abgetrennt. Im Sumpf der Destillationsvorrichtung werden hochpolymere Verbindungen und das zweite deaktivierte Katalysatorsystem (K2) abgetrennt. Die vorliegende Erfindung wird anhand der Figur 1 näher erläutert.

In Figur 1 haben die Bezugszeichen die folgende Bedeutung:
- A: Strom enthaltend das Reaktionsgemisch (R_{G})
- B: Strom enthaltend das erste Gemisch (G1)
- C: Strom enthaltend das zweite Gemisch (G2)
- D: Sumpf der Destillationsvorrichtung
- E: Strom enthaltend das Zielprodukt Cyclododecatrien sowie gegebenenfalls das unpolare Lösungsmittel
- I: Vorrichtung zur Durchführung des Verfahrensschritts a)
- II: Verweilzeitbehälter zur Durchführung des Verfahrensschritts b)
- III: Destillationsvorrichtung zur Abtrennung von Cyclododecatrien gemäß Verfahrensschritt c)

In Figur 1 wird der Strom A, der das Reaktionsgemisch (R_{G}) enthaltend Cyclododecatrien und ein aktives Katalysatorsystem (K) enthält, einem Rührkessel I zugeführt. Über eine Zuleitung wird dem Reaktionsgemisch (R_{G}) in der Vorrichtung I gasförmiger Ammoniak zugeführt. Hierdurch wird das Reaktionsgemisch (R_{G}) in das erste Gemisch (G1) umgewandelt. Das erste Gemisch (G1) enthält Aluminiumverbindungen, die bei 400 °C ein Dampfdruck von größer 1000 mbar aufweisen. Es wird vermutet, dass es sich hierbei um die vorstehend beschriebenen Amido-Aluminiumkomplexe handelt. Aus der Vorrichtung I wird der Strom B enthaltend das erste Gemisch (G1) dem Verweilzeitbehälter II zugeführt. Dem Strom B wird vor Eintritt in den Verweilzeitbehälter II Wasser zugegeben. Im Verweilzeitbehälter II bildet sich aus dem ersten Gemisch (G1) das zweite Gemisch (G2), das keine Aluminiumverbindungen enthält, die bei 400 °C einen Dampfdruck größer 1000 mbar aufweisen. Die Verweilzeit im Verweilzeitreaktor II beträgt mindestens 1 Stunde. Das zweite Gemisch (G2) wird über den Strom C dem Verdampfer III zugeführt. Bevorzugt ist zwischen dem Verweilzeitbehälter II und dem Verdampfer III eine Filtervorrichtung zwischengeschaltet, um gegebenenfalls anfallenden Feststoff vor Eintritt in den Verdampfer III abzutrennen. Aus dem Verdampfer III werden über Kopf als Strom E Cyclododecatrien, gegebenenfalls enthaltene unpolare Lösungsmittel sowie gegebenenfalls weitere flüchtige Nebenprodukte abgetrennt. Das Cyclododecatrien kann gegebenenfalls weiter aufgereinigt werden. Aus dem Sumpf des Verdampfers III werden als Strom D das zweite deaktivierte Katalysatorsystem (K2) sowie hochpolymere und hochsiedende Nebenprodukte abgetrennt.

Die vorliegende Erfindung wird durch die nachfolgenden Beispiele und Vergleichsbeispiele näher erläutert, ohne sie jedoch hierauf zu beschränken.

### Vergleichsbeispiel V1

Unter Inert-Bedingungen werden 4,6 mg Titantetrachlorid (als 1%-ige Lösung in Benzol) und 152 mg Aluminiumsesquichlorid (als 5,48%-ige Lösung in Toluol) in 12,25 g Benzol gelöst. Diese Mischung wird in einen Glas-Autoklaven überführt. Nachfolgend werden bei 70 °C 19,2 ml (entspricht ca. 12 g) 1,3-Butadien über einen Zeitraum von 1 Stunde dem Glas-Autoklaven zugeführt. Hierbei stellt sich Überdruck von 0,4 bar ein.

Nach vollendeter Reaktion wird das so erhaltene Reaktionsgemisch (R_{G}) aus dem Autoklaven entnommen und zweigeteilt. Zu der ersten Hälfte werden 0,15 ml einer 25%-igen wässrigen Ammoniaklösung zugegeben. Zur zweiten Hälfte des Reaktionsgemischs (R_{G}) werden 0,15 ml einer 25%-igen wässrigen Ammoniaklösung und nachfolgend 0,25 g Wasser zugegeben.

In beiden Fällen fällt aus dem Reaktionsgemisch (R_{G}) ein weißer Niederschlag aus und es kommt zur Bildung von zwei Flüssigphasen. Vor der destillativen Abtrennung von Cyclododecatrien muss daher eine Flüssigkeits-Flüssigkeits-Phasentrennungsschritt sowie ein Filtrationsschritt zur Abtrennung des ausgefallenen Feststoffs erfolgen.

Das Vergleichsbeispiel V1 beschreibt ein Verfahren, wie es in der DE 1 768 067 beschrieben ist. Vergleichsbeispiel V1 stellt hierbei die Ausführungsbeispiele 1a und 1b der DE 1 768 067 nach.

### Vergleichsbeispiel V2

In einem 1-Liter-Glasreaktor werden 504 g eines Reaktionsgemischs (R_{G}) vorgelegt, das 57,7 Gew.-% Cyclododecatrien, 34,0 Gew-% Toluol, 0,04 Gew.-% Wasser, 1,8 Gew-% Aluminiumsesquichlorid und 0,14 Gew.-% Titantetrachlorid enthält. Der Rest des Reaktionsgemischs (R_{G}) wird durch hochpolymere Verbindungen, Hochsieder und andere Nebenprodukte gebildet. Zu dem Reaktionsgemisch (R_{G}) werden nachfolgend bei 60 °C 5,2 g gasförmiger Ammoniak zugegeben und das erhaltene Gemisch wird für 4 Stunden gerührt. Hierdurch bildet sich eine leicht trübe Lösung aus. Es fällt jedoch auch nach Abkühlen kein Niederschlag aus. Das so erhaltene Gemisch entspricht dem ersten Gemisch (G1) des erfindungsgemäßen Verfahrens.

Dieses erste Gemisch (G1) wird nachfolgend destillativ aufgearbeitet. Über Kopf wird hierbei Cyclododecatrien und Toluol abgetrennt. In der Destillationsvorrichtung bilden sich nach einer Betriebsdauer von 7 Tagen weiße Ablagerungen aus. Diese weißen Ablagerungen sind in organischen Lösungsmitteln unlöslich und können nur unter großem mechanischem Aufwand entfernt werden.

### Erfindungsgemäßes Beispiel E1

504 g des vorstehend in Vergleichsversuch V2 beschriebenen Reaktionsgemischs (R_{G}) werden in ein Mettler-Toledo-RC1e-Reaktionskalorimeter, ausgestattet mit einem SV01-Glasreaktor (1 Liter), eingebracht. Nachfolgend werden diesem Reaktionsgemisch (R_{G}) bei 60 °C 5,2 g gasförmiger Ammoniak zugegeben und das so erhaltene Gemisch wird für 4 Stunden gerührt. Hierbei wird die Wärmeentwicklung gemessen, die die Bildung des ersten deaktivierten Katalysatorsystems (K1) anzeigt. Die Daten zur Wärmeentwicklung belegen, dass die Bildung des ersten deaktivierten Katalysatorsystems (K1) nach 100 Minuten beendet ist. Das so erhaltene Gemisch entspricht dem ersten Gemisch (G1) des erfindungsgemäßen Verfahrens.

Nachfolgend wird zu diesem ersten Gemisch (G1) bei 60 °C 1 g Wasser zugegeben. Die Wärmeentwicklung der Reaktion wird ebenfalls per Kaloriemetrie verfolgt. Die kalorimetrischen Daten zeigen, dass die Bildung des zweiten Gemischs (G2) nach 133 Minuten beendet ist. Das zweite Gemisch (G2) weist hierbei nur eine einzige Flüssigphase auf, so dass eine Flüssigkeit-Flüssigkeit-Phasentrennung nicht notwendig ist. Darüber hinaus zeigt das zweite Gemisch (G2) nur einen minimalen Niederschlag, der gegebenenfalls abfiltriert werden kann. Das so erhaltene Gemisch entspricht dem zweiten Gemisch (G2) des erfindungsgemäßen Verfahrens.

Das zweite Gemisch (G2) wird nachfolgend einer Destillationsvorrichtung zugeführt, um Cyclododecatrien und Toluol über Kopf abzutrennen.

Auch nach einer Betriebsdauer der Destillationsvorrichtung von 7 Tagen können visuell in der Destillationsvorrichtung keine weißen Ablagerungen festgestellt werden.

Das erfindungsgemäße Beispiel E1 belegt, dass mit dem erfindungsgemäßen Verfahren ein Flüssigkeits-Flüssigkeits-Phasentrennungsschritt nicht erforderlich ist. Zudem fallen bei dem erfindungsgemäßen Verfahren nur minimale Mengen an Feststoff aus. Durch das erfindungsgemäße Verfahren lässt sich zudem die Bildung von Ablagerungen in der Destillationsvorrichtung wirksam verhindern. Dies ermöglicht eine deutlich vereinfachte und damit kostengünstigere Verfahrensführung, sowie einen dauerhaften kontinuierlichen Betrieb zur Aufarbeitung eines Reaktionsgemischs (R_{G}), das Cyclododecatrien enthält.

## Patentansprüche

1. Verfahren zur Aufarbeitung eines Reaktionsgemischs (R_{G}) enthaltend Cyclododecatrien und ein aktives Katalysatorsystem (K), das eine Aluminium-organische Verbindung enthält, umfassend die Schritte:
a) Inkontaktbringen des Reaktionsgemischs (R_{G}) mit gasförmigem Ammoniak unter Erhalt eines ersten Gemischs (G1),
b) Inkontaktbringen des ersten Gemischs (G1) mit Wasser unter Erhalt eines zweiten Gemischs (G2),
c) Destillative Abtrennung von Cyclododecatrien aus dem zweiten Gemisch (G2).

2. Verfahren gemäß Anspruch 1, **dadurch gekennzeichnet, dass** das erste Gemisch (G1) Cyclododecatrien und ein erstes deaktivierte Katalysatorsystem (K1) enthält, wobei das erste Gemisch (G1) Aluminiumverbindungen enthält, die bei 400°C einen Dampfdruck von größer 1000 mbar aufweisen.

3. Verfahren gemäß Anspruch 1 oder 2, **dadurch gekennzeichnet, dass** das zweite Gemisch (G2) Cyclododecatrien und ein zweites deaktiviertes Katalysatorsystem (K2) enthält, wobei das zweite Gemisch (G2) keine Aluminiumverbindungen enthält, die bei 400°C einen Dampfdruck von größer 1000 mbar aufweisen.

4. Verfahren gemäß einem der Ansprüche 1 bis 3, **dadurch gekennzeichnet, dass** das zweite Gemisch (G2) nur eine Flüssigphase enthält.

5. Verfahren gemäß einem der Ansprüche 1 bis 4, **dadurch gekennzeichnet, dass** dem Reaktionsgemisch (R_{G}) in Verfahrensschritt a) pro 1 kg des Reaktionsgemischs (R_{G}) 0,1 bis 20 g gasförmiger Ammoniak zugegeben werden.

6. Verfahren gemäß einem der Ansprüche 1 bis 5, **dadurch gekennzeichnet, dass** dem ersten Gemisch (G1) in Verfahrensschritt b) pro 1 kg des ersten Gemischs (G1) 0,05 bis 1,0 g Wasser zugegeben werden.

7. Verfahren gemäß einem der Ansprüche 1 bis 6, **dadurch gekennzeichnet, dass** das Reaktionsgemisch (R_{G})
10 bis 70 Gew.-% Cyclododecatrien,
10 bis 80 Gew.-% mindestens eines unpolaren Lösungsmittels und
0,5 bis 5 Gew.-% des aktiven Katalysatorsystems (K)
enthält, wobei die Gew.-%-Angaben jeweils auf das Gesamtgewicht des Reaktionsgemischs (R_{G}) bezogen sind.

8. Verfahren gemäß einem der Ansprüche 1 bis 7, **dadurch gekennzeichnet, dass** das aktive Katalysatorsystem (K) mindestens eine Aluminium-organische Verbindung ausgewählt aus der Gruppe bestehend aus Al₂(C₂H₅)₆, Al₂Cl₃(C₂H₅)₃ und AlCl(C₂H₅)₂ sowie mindestens eine Titanverbindung ausgewählt aus der Gruppe bestehend aus Titantetrachlorid und Titanacetylacetonat enthält.

9. Verfahren gemäß einem der Ansprüche 7 bis 8, **dadurch gekennzeichnet, dass** das mindestens eine unpolare Lösungsmittel ausgewählt ist aus der Gruppe bestehend aus Benzol, Cyclohexan, Hexan, Heptan, Octan, Decan und Xylol.

10. Verfahren gemäß einem der Ansprüche 1 bis 9, **dadurch gekennzeichnet, dass** Verfahrensschritt a) für eine Zeitdauer von mindestens 0,1 Stunden durchgeführt wird.

11. Verfahren gemäß einem der Ansprüche 1 bis 10, **dadurch gekennzeichnet, dass** Verfahrensschritt b) für eine Zeitdauer von mindestens 0,5 Stunden durchgeführt wird.

12. Verfahren gemäß einem der Ansprüche 1 bis 11, **dadurch gekennzeichnet, dass** das erste Gemisch (G1) nur eine Flüssigkeitsphase aufweist.

13. Verfahren gemäß einem der Ansprüche 1 bis 12, **dadurch gekennzeichnet, dass** das erste Gemisch (G1) weniger als 1 Gew.-% Feststoff enthält, bezogen auf das Gesamtgewicht des ersten Gemischs (G1).

14. Verfahren gemäß einem der Ansprüche 1 bis 13, **dadurch gekennzeichnet, dass** zweite Gemisch (G2) weniger als 10 Gew.-% Feststoff enthält, bezogen auf das Gesamtgewicht des zweiten Gemischs (G2).

## Claims

1. A process for the work-up of a reaction mixture (R_{M}) comprising cyclododecatriene and an active catalyst system (C) comprising an organoaluminum compound, said process comprising the steps of:
a) contacting the reaction mixture (R_{M}) with gaseous ammonia to obtain a first mixture (M1),
b) contacting the first mixture (M1) with water to obtain a second mixture (M2),
c) distillatively removing cyclododecatriene from the second mixture (M2).

2. The process according to claim 1 wherein the first mixture (M1) comprises cyclododecatriene and a first deactivated catalyst system (C1) wherein the first mixture (M1) comprises aluminum compounds having a vapor pressure of more than 1000 mbar at 400°C.

3. The process according to either of claims 1 and 2 wherein the second mixture (M2) comprises cyclododecatriene and a second deactivated catalyst system (C2) wherein the second mixture (M2) comprises no aluminum compounds having a vapor pressure of more than 1000 mbar at 400°C.

4. The process according to any of claims 1 to 3 wherein the second mixture (M2) comprises only one liquid phase.

5. The process according to any of claims 1 to 4 wherein process step a) comprises adding to the reaction mixture (R_{M}) 0.1 to 20 g of gaseous ammonia per 1 kg of the reaction mixture (R_{M}).

6. The process according to any of claims 1 to 5 wherein process step b) comprises adding to the first mixture (M1) 0.05 to 1.0 g of water per 1 kg of the first mixture (M1).

7. The process according to any of claims 1 to 6 wherein the reaction mixture (R_{M}) comprises
10% to 70% by weight of cyclododecatriene
10% to 80% by weight of at least one apolar solvent and
0.5% to 5% by weight of the active catalyst system (C),
wherein the % by weight values are in each case based on the total weight of the reaction mixture (R_{M}).

8. The process according to any of claims 1 to 7 wherein the active catalyst system (C) comprises at least one organoaluminum compound selected from the group consisting of Al₂(C₂H₅)₆, Al₂Cl₃(C₂H₅)₃ and AlCl(C₂H₅)₂ and at least one titanium compound selected from the group consisting of titanium tetrachloride and titanium acetylacetonate.

9. The process according to either of claims 7 and 8 wherein the at least one apolar solvent is selected from the group consisting of benzene, cyclohexane, hexane, heptane, octane, decane and xylene.

10. The process according to any of claims 1 to 9 wherein process step a) is carried out over a period of at least 0.1 hours.

11. The process according to any of claims 1 to 10 wherein process step b) is carried out over a period of at least 0.5 hours.

12. The process according to any of claims 1 to 11 wherein the first mixture (M1) comprises only one liquid phase.

13. The process according to any of claims 1 to 12 wherein the first mixture (M1) comprises less than 1% by weight of solid based on the total weight of the first mixture (M1).

14. The process according to any of claims 1 to 13 wherein the second mixture (M2) comprises less than 10% by weight of solid based on the total weight of the second mixture (M2).

## Revendications

1. Procédé de traitement d'un mélange réactionnel (R_{G}) contenant du cyclododécatriène et un système catalytique actif (K), qui contient un composé organique d'aluminium, comprenant les étapes suivantes :
a) la mise en contact du mélange réactionnel (R_{G}) avec de l'ammoniac gazeux pour obtenir un premier mélange (G1),
b) la mise en contact du premier mélange (G1) avec de l'eau pour obtenir un deuxième mélange (G2),
c) la séparation par distillation de cyclododécatriène du deuxième mélange (G2).

2. Procédé selon la revendication 1, **caractérisé en ce que** le premier mélange (G1) contient du cyclododécatriène et un premier système catalytique désactivé (K1), le premier mélange (G1) contenant des composés d'aluminium qui présentent à 400 °C une pression de valeur supérieure à 1 000 mbar.

3. Procédé selon la revendication 1 ou 2, **caractérisé en ce que** le deuxième mélange (G2) contient du cyclododécatriène et un deuxième système catalytique désactivé (K2), le deuxième mélange (G2) ne contenant pas de composés d'aluminium qui présentent à 400 °C une pression de valeur supérieure à 1 000 mbar.

4. Procédé selon l'une quelconque des revendications 1 à 3, **caractérisé en ce que** le deuxième mélange (G2) contient uniquement une phase liquide.

5. Procédé selon l'une quelconque des revendications 1 à 4, **caractérisé en ce que** 0,1 à 20 g d'ammoniac gazeux est ajouté au mélange réactionnel (R_{G}) à l'étape de procédé a) pour 1 kg du mélange réactionnel (R_{G}).

6. Procédé selon l'une quelconque des revendications 1 à 5, **caractérisé en ce que** 0,05 à 1,0 g d'eau est ajoutée au premier mélange (G1) à l'étape de procédé b) pour 1 kg du premier mélange (G1).

7. Procédé selon l'une quelconque des revendications 1 à 6, **caractérisé en ce que** le mélange réactionnel (R_{G}) contient
10 à 70 % en poids de cyclododécatriène,
10 à 80 % en poids d'au moins un solvant apolaire et
0,5 à 5 % en poids du système catalytique actif (K),
les données de % en poids se rapportant à chaque fois au poids total du mélange réactionnel (R_{G}).

8. Procédé selon l'une quelconque des revendications 1 à 7, **caractérisé en ce que** le système catalytique actif (K) contient au moins un composé organique d'aluminium choisi dans le groupe constitué par Al₂(C₂H₅)₆, Al₂Cl₃(C₂H₅)₃ et AlCl(C₂H₅)₂, ainsi qu'au moins un composé de titane choisi dans le groupe constitué par le tétrachlorure de titane et l'acétylacétonate de titane.

9. Procédé selon l'une quelconque des revendications 7 à 8, **caractérisé en ce que** ledit au moins un solvant apolaire est choisi dans le groupe constitué par le benzène, le cyclohexane, l'hexane, l'heptane, l'octane, le décane et le xylène.

10. Procédé selon l'une quelconque des revendications 1 à 9, **caractérisé en ce que** l'étape de procédé a) est réalisée pendant une durée d'au moins 0,1 heure.

11. Procédé selon l'une quelconque des revendications 1 à 10, **caractérisé en ce que** l'étape de procédé b) est réalisée pendant une durée d'au moins 0,5 heure.

12. Procédé selon l'une quelconque des revendications 1 à 11, **caractérisé en ce que** le premier mélange (G1) comprend uniquement une phase liquide.

13. Procédé selon l'une quelconque des revendications 1 à 12, **caractérisé en ce que** le premier mélange (G1) contient moins de 1 % en poids de solide, par rapport au poids total du premier mélange (G1).

14. Procédé selon l'une quelconque des revendications 1 à 13, **caractérisé en ce que** le deuxième mélange (G2) contient moins de 10 % en poids de solide, par rapport au poids total du deuxième mélange (G2).
